Europäisches Patentamt

**European Patent Office**

**Office européen des brevets**

(11) Publication number: **0 047 735**
**B1**

(12) **EUROPEAN PATENT SPECIFICATION**

(45) Date of publication of patent specification: **06.05.87**

(21) Application number: **80902060.5**

(22) Date of filing: **03.11.80**

(86) International application number:
**PCT/GB80/00191**

(87) International publication number:
**WO 81/01238 14.05.81 Gazette 81/12**

(51) Int. Cl.⁴: **A 61 B 5/14, B 01 L 3/14**

(54) **BLOOD SAMPLING SETS.**

(30) Priority: **02.11.79 GB 7938039**
**29.02.80 GB 8006866**

(43) Date of publication of application:
**24.03.82 Bulletin 82/12**

(45) Publication of the grant of the patent:
**06.05.87 Bulletin 87/19**

(84) Designated Contracting States:
**AT CH DE FR LI LU NL SE**

(56) References cited:
**US-A-3 930 413**
**US-A-3 965 889**
**US-A-4 092 113**
**US-A-4 166 450**

(73) Proprietor: **Labco Limited**
**Unit 3 Crusader Industrial Estate Halifax Road**
**Cressex Industrial Estate**
**High Wycombe, Bucks. HP12 3SD (GB)**

(72) Inventor: **Cook, Ivan John Yason**
**Greenlauds Teignmonth Road**
**Maidencomhe Torquay Devon (GB)**

(74) Representative: **Simpson, Ronald Duncan Innes**
**et al**
**A.A.Thornton & Co. Northumberland House**
**303-306 High Holborn**
**London WCIV 7LE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to blood sampling vials of the type comprising a sealed vial, usually of transparent or translucent glass or plastics, whose interior is partially evacuated, for use with a holder comprising a barrel having a double ended hypodermic needle carried on one end of the barrel.

In use, one end of the hypodermic needle is inserted into the patient, and the other end is caused to pierce a seal in the vial, which is guided and supported by the barrel, so that blood is drawn into the vial to satisfy the partial vacuum.

In known equipment of this type, the vial is usually sealed by an internal plug of elastomeric material and such sealed vials are satisfactory during blood sampling and in storage and transit of the sealed vial with its blood sample.

However, problems can and do arise at the blood testing stage when the vial is opened for access to the blood sample. Due to changes in internal volume which are unavoidable when removing an internal plug, small droplets of blood are inevitably ejected from the vial and fall to the work surface. Additionally, blood adhering to the plug often drops to the work surface causing risk of contamination by pathogens. An "aerosol effect" is also said to occur when the internal plug is removed in which finely dispersed droplets of blood ejected into the atmosphere may be inhaled causing risk of transmission of disease. It is also difficult to remove the internal plug by hand without contaminating the fingers and the surface onto which the plug is placed.

The present invention aims at the provision of a vial and closure for use in a blood sampling set of the general type first described, in which the closure obviates or reduces the above mentioned risk of contamination.

A solution to this problem has been proposed in U.S. Patent 4166450, in which a face-sealing member (or liner) is provided to seal the vial.

By comparison with the said U.S. Specification, the present invention provides a method of evacuating and assembling the vial and closure assembly which avoids the need to perforate the sealing liner prior to use in blood sampling, and the partially evacuated vial is of simplified construction.

The invention accordingly provides a method of assembling and evacuating a blood sampling vial and closure, adapted for use with a holder carrying a double ended needle, the closure comprising a liner formed as a disc of elastomeric material fitting within a closure cap of inverted cup shape having a central hole in its transverse end wall, characterised in that the liner is located loosely over the mouth of the vial, within a chamber which is then evacuated to permit partial evacuation of the vial and then repressurising the chamber, whereby the pressure difference causes the liner to seal against the mouth of the vial and thereby retain the partial vacuum, and the cap is then securely but removably engaged by its side wall over the neck of the vial to clamp the periphery of the liner directly against the mouth of the vial, the said liner thereafter being securely retained by its periphery in the cap, for eventual removal with the cap from the vial.

The invention also provides a partially evacuated blood sampling vial and closure assembly, for use with a holder carrying a double ended needle, the closure comprising a removable cap of inverted cup shape in secure but releasable engagement over the neck of the vial, and a liner formed as a disc of elastomeric material located within the cap against its transverse end wall, and in sealing engagement against the mouth of the vial, the cap having a central hole through its end wall to permit the passage of the inner end of the needle through the end wall and liner in use of the vial, characterised in that the liner is imperforate, ans seats directly against the mouth of the vial, and is retained by its periphery in the cap on removal of the cap from the vial.

One form of vial and closure assembly and method of evactuating and assembling it, both in accordance with the invention, are described below, by way of example, with reference to the accompanying drawings in which:

Figure 1 is a part sectional elevation of the closure member of the vial;

Figure 2 is a side view of the assembly in use.

The illustrated closure member comprises a cap 1 which is preferably moulded of a rigid plastics material, and has a simple inverted cup shape with an internally screw-threaded side wall 2 and a top wall 3 having a central hole 4. The cap 1 carries a liner 7 formed as a disc of rubber or other elastomeric material which is fitted in the upper region of the cap against the underside of the top wall.

The cap is applied to a vial 12 having a screw-threaded neck or lug (annular ring) and the cap holds the liner in sealing engagement with the mouth of the vial.

The liner has a downwardly projecting plug section having a chamfered side wall 8 to assist in pre-location of the liner, and the periphery 9 of the liner is chamfered upwardly and inwardly to assist its location and retention in the cap 1.

With this construction, the liner is first loosely located over the mouth of the vial, the plug section assisting in centering the liner, and a batch of vials with loose liners is placed in a vacuum chamber. On evacuation of the chamber, air is extracted from the interior of each vial, the liner lifting slightly to permit the escape of air. When the chamber is re-pressurised, the pressure difference causes the liner to seal against the mouth of the vial and thereby retain the partial vacuum.

The evacuated vials are then removed from the pressure chamber and the screw caps are applied with slight interference between the screw threads of the cap and the chamfered edge of the liner, which is slightly compressed in the process. When the cap is screwed fully home, the liner is firmly engaged in the cap and after taking a blood sample, the cap is unscrewed, retaining the liner in position where it is shielded from accidental contact by the side wall of the cap.

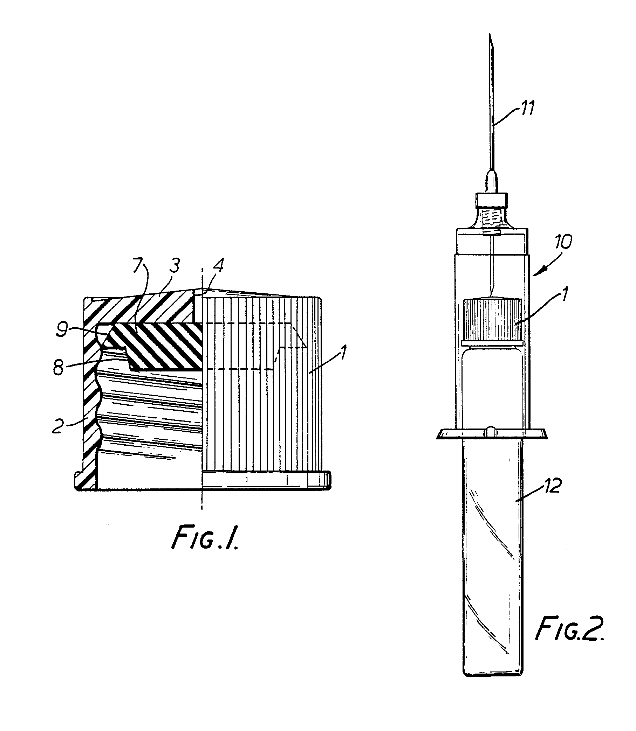

The chamfered design shown in Figure 1 has been found extremely effective in ensuring that the liner is retained to the cap on opening the vial, the slight compression and consequent frictional grip being adequate for this purpose since at the time of opening the vial, following blood sampling, the interior of the vial is at atmospheric pressure. However, if a more positive retention were required, the cap may have an undercut groove on the base of the screw thread or a narrow annular rib may be provided to trap the periphery of the liner.

The closure member, instead of taking the form of a screw cap, may for example take the form of a snap-on or crimped lid retained by an annular ring of suitable section on the outside of the vial neck. If the cap is screw threaded or snap-fitting, a tamper proof capsule may be applied to provided a visual indication of possible loss of vacuum or sterility of the vial.

For the sake of completeness, a blood sampling set is illustrated in Figure 2. It comprises a holder in the form of a standard barrel 10 open at one end and fitted at the opposite end with a double-ended needle 11, and the vial 12 fitting easily in the bore of the barrel 10 and having its inner end closed by a cap 1 and liner of any of the forms described and/or illustrated herein. The needle 11 may be valved or unvalved, in known manner.

The vial is used in conventional manner to take a blood sample and transferred to the laboratory where the cap is removed to permit access to the sample. The droplet and "aerosol effect" is obviated since the face-to-face seal between the liner and the vial is gently and easily broken by unscrewing or otherwise releasing the cap. This is in contrast to the removal of a conventional internal plug which involves alteration in the internal volume of the space above the surface of the blood and a sudden inrush of air when the seal is finally and abruptly broken.

Furthermore, the drop of blood which will usually adhere to the interior of the liner is shielded from contact by the side wall 2 of the cap.

## Claims

1. A method of assembling and evacuating a blood sampling vial and closure, adapted for use with a holder carrying a double-ended needle, the closure comprising a liner (7) formed of a disc of elastomeric material fitting within a closure cap (1) of inverted cup shape having a central hole (4) in its transverse end wall, characterised in that the liner (7) is located loosely over the mouth of the vial (12), within a chamber which is then evacuated to permit partial evacuation of the vial and then repressurising the chamber, whereby the pressure difference causes the liner to seal against the mouth of the vial and thereby retain the partial vacuum, and the cap (1) is then securely but removably engaged by its side wall (2) over the neck of the vial to clamp the periphery (9) of the liner directly aganst the mouth of the vial, the said liner thereafter being securely retained by its periphery (9) in the cap (1), for eventual removal with the cap from the vial.

2. A partially evacuated blood sampling vial and closure assembly, for use with a holder carrying a double ended needle (11), the closure comprising a removable cap (1) of inverted cup shape in secure but releasable engagement over the neck of the vial (12), and a liner (7) formed as a disc of elastomeric material located within the cap (1) against its transverse end wall, and in sealing engagement against the mouth of the vial, the cap having a central hole (4) through its end wall to permit the passage of the inner end of the needle (11) through the end wall and liner in use of the vial, characterised in that the liner (7) is imperforate, and seats directly against the mouth of the vial, and is retained by its periphery (9) in the cap on removal of the cap from the vial.

3. A vial and closure assembly according to claim 2, characterised in that the liner has a plug section (8) formed· on its inner surface for centralising the liner relative to the mouth of the vial.

## Patentansprüche

1. Verfahren zum Zusammensetzen und Evakuieren eines Blutprobenglasfläschchens mit Verschluß, das zur Verwendung mit einem Halter eingerichtet ist, der eine an beiden Enden scharfe Nadel trägt, wobei der Verschluß eine Unterlage (7) aus einer Scheibe aus elastomeren Material enthält, die in einer Verschlußkappe (1) von umgekehrt becherförmiger Gestalt sitzt, die ein Mittenloch (4) in ihrer sich quererstreckenden Stirnwand aufweist, dadurch gekennzeichnet, daß die Unterlage (7) lose über der Mündung des Glasfläschchens (12) innerhalb einer Kammer gelegen ist, die anschließend evakuiert wird, um eine Teilevakuierung des Glasfläschchens und eine anschließende Wiederunterdrucksetzung der Kammer zu ermöglichen, wodurch die Druckdifferenz bewirkt, daß die Unterlage dichtend auf der Mündung des Glasfläschchens aufliegt und dadurch das Teilvakuum aufrechterhält, und daß die Kappe (1) dann sicher, jedoch abnehmbar mit ihrer Seitenwand (2) den Hals des Glasfläschchens umgreift, um den Umfang (9) Unterlage direkt gegen die Mündung des Glasfläschchens zu klemmen, wobei die genannte Unterlage anschließend sicher an ihrem Umfang (9) in der Kappe (1) festgehalten wird, um ggf. zusammen mit der Kappe von dem Glasfläschchen abgenommen zu werden.

2. Teilevakuiertes Blutprobenglasfläschchen mit Verschluß zur Verwendung mit einem Halter, der eine an beiden Enden scharfe Nadel (11) trägt, wobei der Verschluß eine abnehmbare Kappe (1) von umgekehrt becherförmiger Gestalt aufweist, die fest, aber abnehmbar den Hals des Glasfläschchens (12) umgreift, und weiterhin eine Unterlage (7) in Form einer Scheibe aus elastomeren Material enthält, die innerhalb der Kappe (1) gegen deren querlaufende Stirnwand gelegen ist und in dichtendem Eingriff mit der Mündung des Glasfläschchens ist, wobei die Kappe ein Mittenloch (4) durch ihre Stirnwand aufweist, durch das das innere Ende der Nadel (11) durch die Endwand und die Unterlage im Gebrauch des Glasfläschchens hindurchtreten kann, dadurch gekennzeichnet, daß die Unterlage (7) geschlossen ist und direkt auf der Mündung des Glasfläschchens sitzt und von ihrem Umfang (9) in der Kappe beim Abnehmen der Kappe von dem Glasfläschchen festgehalten ist.

3. Glasfläschchen mit Verschluß nach Anspruch 2, dadurch gekennzeichnet, daß die Unterlage einen Stopfenabschnitt (8) aufweist, der an ihrer Innenseite ausgebildet ist, um die Unterlage in bezug auf die Mündung des Glasfläschchens zu zentrieren.

**Revendications**

1. Procédé d'assemblage et de mise sous vide d'une fiole d'échantillonnage de sang et d'une fermeture de fiole, adaptées pour être utilisées avec un support portant une aiguille à deux extrémités pointues, la fermeture étant constituée d'une garniture (7) formée d'un disque d'élastomère s'ajustant dans un bouchon (1) en forme de godet retourné et possédant un trou central (4) dans son fond, caractérisé en ce qu'il consiste à placer la garniture (7) librement sur l'embouchure de la fiole (12) à l'intérieur d'une chambre dans laquelle on fait ensuite la vide pour permettre la mise sous vide partielle de la fiole, à rétablir ensuite la pression dans la chambre, de sorte que la différence de pression provoque l'application étanche de la garniture contre l'embouchure de la fiole et que, ainsi, le vide partiel dans la fiole est maintenu, puis à fixer le bouchon (1) ferement mais de façon amovible par sa paroi latérale (2) sur le col de la fiole de manière que la périphérie (9) de la garniture soit pressée directement contre l'embouchure de la fiole, la garniture étant ensuite retunue fermement par sa périphérie (9) dans le bouchon (1), de sorte qu'elle est enlevée avec le bouchon lorsque celui-ci est retiré de la fiole par la suite.

2. Ensemble formé d'une fiole d'échantillonnage de sang partiellement mise sous vide et d'une fermeture, destiné à être utilisé avec un support portant une aiguille (11) à deux extrémités pointues, la fermeture étant constituée d'un bouchon (1) amovible en forme de godet retourné, qui est fixé fermement mais de façon amovible sur le col de la fiole (12), ainsi que d'une garniture (7) formée d'un disque d'élastomère disposé dans le bouchon (1) contre le fond de celui-ci, et appliqué étanche contre l'embouchure de la fiole, le bouchon possédant un trou central (4) dans son fond pour permettre le passage de l'extrémité intérieure de l'aiguille (11) à travers ce fond et au travers de la garniture lors de l'utilisation de la fiole, caractérisé en ce que la garniture (7) n'est pas perforée et est appliquée directement contre l'embouchure de la fiole et qu'elle est retenue par sa périphérie (9) dans le bouchon lorsque le bouchon est retiré de la fiole.

3. Ensemble selon la revendication 2, caractérisé en ce que la garniture comporte sur sa surface interne une portion à emboîtement (8) pour centrer la garniture par rapport à l'embouchure de la fiole.

FIG.1.

FIG.2.